Europäisches Patentamt

European Patent Office

Office européen des brevets

(11) Veröffentlichungsnummer: **0 232 501 B1**

# EUROPÄISCHE PATENTSCHRIFT

(45) Veröffentlichungstag der Patentschrift: **15.04.92**

(21) Anmeldenummer: **86116933.2**

(22) Anmeldetag: **05.12.86**

Die Akte enthält technische Angaben, die nach
dem Eingang der Anmeldung eingereicht wurden
und die nicht in dieser Patentschrift enthalten sind.

(51) Int. Cl.5: **A61K 35/78**, A61K 35/60,
A61K 31/23

(54) **Pharmazeutisches Präparat zur Behandlung von Störungen des Fettstoffwechsels (Hyperlipoproteinämien).**

(30) Priorität: **19.12.85 LU 86220**

(43) Veröffentlichungstag der Anmeldung:
**19.08.87 Patentblatt 87/34**

(45) Bekanntmachung des Hinweises auf die
Patenterteilung:
**15.04.92 Patentblatt 92/16**

(84) Benannte Vertragsstaaten:
**AT CH DE FR GB IT LI**

(56) Entgegenhaltungen:
**DE-A- 2 305 553**
**DE-C- 820 950**
**DE-C- 943 250**
**GB-A- 1 011 265**

PATENT ABSTRACTS OF JAPAN, Band 7, Nr.
155 (C-175)[1300], 07 Juli 1983

PATENT ABSTRACTS OF JAPAN, Band 7, Nr.
28 (C-149)[1173], 04 Februar 1983

PATENT ABSTRACTS OF JAPAN, Band 10,
Nr. 128 (C-345)[2185], 13 Mai 1986

ROTE LISTE, 1984, Nr. 42046, Gelovitall

(73) Patentinhaber: **CHIMICASA GMBH**
**Wiesentalstrasse 81**
**CH-7000 Chur(CH)**

(72) Erfinder: **Deininger, Rolf, Dr.**
**Fürst Pückler-Str. 44**
**W-5000 Köln 41(DE)**

(74) Vertreter: **Hach, Hans Karl, Dr.**
**Tarunstrasse 23**
**W-6950 Mosbach-Waldstadt(DE)**

EP 0 232 501 B1

**Beschreibung**

Die Erfindung betrifft ein pharmazeutisches Präparat zur Behandlung von Störungen des Fettstoffwechsels (Hyperlipoproteinämien) oder Beutlipidsenkendes pharmazeutisches Präparat, das als Wirkstoff Knoblauchextrakt und hochungesättigte Fettsäuren vom Omega-3-Typ, darunter auch Eicosapentaensäure, und als Trägersubstanz Öl enthält.

Ein solches Präparat ist aus Rote Liste 1984, Nr. 42046 bekannt.

Der Knoblauchextrakt enthält als therapeutisch wirksame Verbindungen das instabile Alliin und dessen Umwandlungsprodukte Allicin, 3-Vinyl-1,2-dithiin und Ajoen. Diese Verbindungen bewirken eine Verringerung erhöhter Serumcholesterinwerte und Steigerung der HDL-Lipidfraktion und Hemmung der Thrombozytenaggregation, und Hemmung der Prostaglandin-Biosynthese.

Erhöhte Cholesterinwerte, verringerte HDL-Werte und gesteigerte Affinität der Thrombozytenaggregation sind Risikofaktoren des Stoffwechsels für die Entstehung der Arteriosklerose und des Infarkts.

Aufgabe der Erfindung ist es, die pharmazeutische Wirkung des Präparates zu verstärken.

Die Erfindung ist dadurch gekennzeichnet, daß es bezogen auf Knoblauchextrakt, der aus 100 Gewichtsteilen frischem Knoblauch erhalten wurde, 50 bis 200, vorzugsweise 100, Gewichtsteile Fischöl, das aus Fischfleisch von Kaltwasserfischen gewonnen wurde, und hochungesättigte Fettsäuren vom Omega-3-Typ aufweist, enthält und daß das eingesetzte Fischöl 15 bis 30 Gewichtsprozent Eicosapentaensäure enthält.

Bei den hochungesättigten Fettsäuren vom Omega-3-Typ handelt es sich beispielsweise um Eicosapentaensäure und Decosahexaensäure. Hochungesättigte Fettsäuren wirken senkend auf den Triglyceridgehalt und hemmen die Thrombozytenaggregation, verstärken mithin wesentliche Wirkungen des Knoblauchextraktes.

Diese pharmazeutische Wirkung des Fischöls hat man bei Lebertran und Fischölen mit Tagesdosen ab 10 g (Gramm) - 20 g für die erwachsene Person beobachtet. In Verbindung mit dem Knoblauchextrakt zeigte sich die pharmazeutische Wirkung des Fischöls bereits bei niedrigen Tagesdosen von 2 - 4 g für die erwachsene Person. Das ist bedeutungsvoll, weil eine Wirksamkeitsverstärkung eintritt bei verbesserter Compliance und weil mit zunehmender Dosierung Patienten die Einnahme von Fischölpräparaten ablehnen wegen der damit verbundenen Geruchabsonderung. Eine solche bei hohen Tagesdosen von 10 g und mehr unvermeidliche Geruchsabsonderung wird bei Tagesdosen von 3,5 g fast vollständig vermieden. Die Erfindung kommt mit stark reduzierter Dosierung aus, die bedingt ist durch das Zusammenwirken der Triglyceridsenkenden Wirkung des Fischöls und der Cholesterinsenkenden Wirkung des Knoblauchs. Das sind gleichsinnige Wirkungen auf die Risikofaktoren der Arteriosklerose und des Infarkts.

Auf Knoblauchextrakt aus 100 Gewichtsteilen frischem Knoblauch werden zweckmäßig 0,5 bis 2,0, vorzugsweise 1,3, Gewichtsteile Vitamin E oder Eilecithin eingesetzt zur Stabilisierung, insbesondere als Oxydationsschutz.

Vorzugsweise wird Fischöl eingesetzt, das einen Gehalt von 18 bis 50 Gewichtsprozent, vorzugsweise 22 bis 31 Gewichtsprozent, an hochungesättigten Fettsäuren vom Omega-3-Typ enthält. Dabei handelt es sich vorzugsweise um Fischöl, das 15 bis 20 Gewichtsprozent Eicosapentaensäure (2o : 5 Omega-3) und 7 bis 20 Gewichtsprozent, vorzugsweise 10 bis 13 Gewichtsprozent, Docosahexaensäure (22 : 6 Omega-3) enthält.

Vorzugsweise wird Fischöl eingesetzt, das aus dem Fleisch von Lachsen, Heringen, Salmen, Forellen, Sardellen (Menarden), Makrelen und/oder Sardinen gewonnen ist.

Vorzugsweise wird ein Knoblauchextrakt eingesetzt, der aus kleingeschnittenem, getrocknetem, vorzugsweise gefriergetrocknetem Knoblauch gewonnen ist. Die Gefriertrocknung empfiehlt sich, weil sie schonend erfolgt und die Wirkstoffe unbeschädigt läßt.

Es kann ein Knoblauchextrakt eingesetzt werden, der durch Anwendung organischer Lösungsmittel, zum Beispiel Methylalkohol oder Äthylalkohol, aus frischem, kleingeschnittenen Knoblauch gewonnen ist.

Es kann auch, wie bei bekannten Knoblauchpräparaten, Knoblauchextrakt eingesetzt sein, der durch Mazerisierung von frischem, kleingeschnittenen Knoblauch mit therapeutisch unwirksamem Öl, zum Beispiel Rüböl, und anschließende Extraktion gewonnen ist. Dazu wird frischer Knoblauch mit einer Teilchengröße von 1 bis 3 mm (Millimeter) kleingeschnitten und und dann in das zur Mazerisierung eingesetzte Öl eingemischt und intensiv verrührt. Der ölige Knoblauchextrakt wird dann durch Filtration von den festen Knoblauchrückständen getrennt.

Der Einsatz von mit Öl aus geschnittenem frischen Knoblauch gewonnenen Knoblauchwirkstoffen ist auch vorteilhaft, weil die Wirkstoffe des frischen Knoblauchs in dieser Weise extrahiert über mehr als drei Jahre stabil bleiben. Das gleiche gilt auch, wenn man gefriergetrockneten Knoblauch einsetzt oder extrahierte Knoblauchwirkstoffe einsetzt, die aus getrocknetem Knoblauch durch Extraktion mit organischen Lösungsmitteln gewonnen wurden.

Bevorzugt eingesetzt ist ein Knoblauchextrakt, der durch Mazerisieren von frischem, kleingeschnittenen Knoblauch mit dem Fischöl gewonnen ist.

Durch den Einsatz des Fischöls nicht nur als Wirksubstanz, sondern auch als Mazerationsmittel, kann der Einsatz eines anderen Extraktionsmittels, zum Beispiel des Rüböls als Hilfsstoff zur Mazerisierung, vermieden werden. Außerdem vermeidet man notwendige Maßnahmen, das Rüböl aus dem Präparat zu entfernen, beziehungsweise man vermeidet unerwünschte Beimengungen von Rüböl im Präparat. Zudem kann nicht ohne weiteres ausgeschlossen werden, daß von dem Rüböl oder einem anderen Extraktionsmittel unerwünschte Substanzen, wie zum Beispiel beim Rüböl Erucasäure, in den Extrakt übergehen.

Verwendet man Fischöl zur Mazerisierung, dann empfiehlt es sich, das Fischöl in demjenigen Mengenverhältnis zum Knoblauch zur Mazerisierung einzusetzen, in dem es nach der vorgenommenen Mazeration im Präpart vorliegen soll. Dabei kann man für die Mazeration den Fischöleinsatz auf das Minimum beschränken, das notwendig ist, um die Knoblauchwirkstoffe mit der gewünschten Ausbeute herauszulösen. Man kann aber auch eine größere Menge Fischöl für die Mazeration einsetzen.

Im Fall man einen geringeren Fischölanteil als den bei der Mazerisierung anfallenden wünscht, kann man die Mazeration mit einem so geringen Fischölanteil durchführen, daß gerade der Knoblauchextrakt mit der gewünschten Ausbeute gewonnen wird, und anschließend der bei der Mazeration gewonnenen Lösung zusätzlichen Knoblauchextrakt zusetzen, der auf andere Weise gewonnen ist.

Das Präparat wird vorzugsweise dargereicht in Form von Kapseln, wobei jede Kapsel 500 bis 1000, vorzugsweise 750, mg (Milligramm) Fischöl und die entsprechenden Anteile an Knoblauchextrakt und Vitamin E enthält.

Das Präparat kann auch vorteilhaft in flüssiger Form dargereicht werden.

Zur Therapie werden für eine 70 kg (Kilogramm) schwere erwachsene Person 2 bis 10 g (Gramm) des Präparates pro Tag verabfolgt. Es hat sich gezeigt, daß man in den meisten Fällen hervorragende Ergebnisse erzielt bei Dosierungen von 2 bis 4 g pro Tag.

Beispiel 1

1000 g Makrelenöl mit einem Gehalt von 30 % (Prozent) Eicosapentaensäure und 20 % Docosahexaensäure werden mit 700 g geschältem, frischem, in eine Teilchengröße von 2 bis 3 mm zerschnittenem Knoblauch und Zugabe von 14 g Vitamin E vermischt und 6 Stunden bei Zimmertemperatur intensiv verrührt. Dann werden die Knoblauchrückstände abfiltriert.

Die so gewonnene ölige Lösung wird dann in Portionen zu 700 mg auf Kapseln abgefüllt.

Zur Therapie werden 3 bis 6 Kapseln pro Tag einem 70 kg schweren Erwachsenen verabfolgt, das entspricht 3,1 g öliger Lösung.

Weitere Beispiele 2 und 3 unterscheiden sich von dem Beispiel 1 nur durch die aus der nachfolgenden Tabelle ersichtlichen Angaben.

## TABELLE 1

| Beispiel | 2 | 3 |
|---|---|---|
| A | Öl aus ganzen Heringen | Öl aus ganzen Lachsen |
| B | 1000 | 1000 |
| C | 17 | 15 |
| D | 11 | 7 |
| E | 1000 | 500 |
| F | 13 | 13 |
| G | – | – |
| H | 2,1 | 3,3 |

In der Tabelle bedeutet:

A = Art des eingesetzten Fischöls

B = Menge eingesetztes Fischöl in g

C = Prozentgehalt an Eicosapentaensäure

D = Prozentgehalt an Docosahexaensäure

E = Menge eingesetzter Knoblauch in g

F = Menge eingesetztes Vitamin E in g

G = Menge eingesetztes Eilecithin in g

H = Dosierung für einen 70 kg schweren Erwachsenen täglich in g

Beispiel 4

1000 g geschälter, frischer, in eine Teilchengröße von 2 bis 3 mm zerschnittener Knoblauch wird gefriergetrocknet. Der so getrocknete Knoblauch wird 1000 g Öl, das aus ganzen Sardinen gewonnen ist, zugesetzt und 12 Stunden bei Zimmertemperatur verrührt. Das eingesetzte Öl aus ganzen Sardinen enthält 18 - 20 % Eicosapentaensäure und 12 % Docosahexaensäure. Die Knoblauchrückstände aus der Mischung werden abfiltriert.

Der so gewonnenen öligen Lösung werden zugesetzt 13 g Vitamin E und 20 g Eilecithin. Die ölige Lösung wird dann in Portionen zu 700 mg auf Kapseln abgefüllt. Zur Therapie werden 3 bis 6 Kapseln pro Tag einem 70 kg schweren Erwachsenen verabfolgt. Das entspricht 2,1 bis 4,2 g öliger Lösung pro Tag.

Beispiel 5

Wie Beispiel 4 mit dem einzigen Unterschied, daß anstelle des gefriergetrockneten Knoblauchs Knoblauchextrakt eingesetzt wird. Dieser Knoblauchextrakt wird gewonnen, indem 1000 g geschälter, frischer, in eine Teilchengröße von 2 bis 3 mm zerschnittener Knoblauch mit 2000 g Methylalkohol versetzt und bei Zimmertemperatur 6 Stunden lang verrührt wird. Von dieser Mischung werden anschließend die festen Bestandteile abfiltriert und der Äthylalkohol wird im Vakuum zur Trockne abdestilliert, so daß das Knoblauchextrakt zurückbleibt.

Abänderung der Beispiele 1 bis 5

Statt der Darreichung in Form von Kapseln wird das Präparat als Flüssigkeit dargereit und zwar zur Einnahme pro Tag für einen 70 kg schweren Erwachsenen 2 is 4 g öliger Lösung aus den genannten Beispielen.

**Patentansprüche**

1. Pharmazeutisches Präparat zur Behandlung von Störungen des Fettstoffwechsels (Hyperlipoproteinämien), das als Wirkstoff Knoblauchextrakt und hochungesättigte Fettsäuren vom Omega-3-Typ, darunter auch Eicosapentaensäure, und als Trägersubstanz Öl enthält, dadurch gekennzeichnet,

daß es bezogen auf Knoblauchextrakt, der aus 100 Gewichtsteilen frischem Knoblauch erhalten wurde, 50 bis 200, vorzugsweise 100, Gewichtsteile Fischöl, das aus Fischfleisch von Kaltwasserfischen gewonnen wurde, und hochungesättigte Fettsäuren vom Omega-3-Typ aufweist, enthält und

daß das eingesetzte Fischöl 15 bis 30 Gewichtsprozent Eicosapentaensäure enthält.

2. Präparat nach Anspruch 1, dadurch gekennzeichnet,

daß 0,5 bis 2,0, vorzugsweise 1,3, Gewichtsteile Vitamin E auf Knoblauchextrakt aus 100 Gewichtsteilen frischem Knoblauch eingesetzt sind.

3. Präparat nach Anspruch 1, dadurch gekennzeichnet,

daß das eingesetzte Fischöl 18 bis 50 Gewichtsprozent, vorzugsweise 22 bis 31 Gewichtsprozent, hochungesättigte Fettsäuren vom Omega-3-Typ enthält.

4. Präparat nach Anspruch 1, dadurch gekennzeichnet,

daß das eingesetzte Fischöl enthält

7 bis 20 Gewichtsprozent, vorzugsweise

10 bis 13 Gewichtsprozent, Docosahexaensäure.

5. Präparat nach Anspruch 1, dadurch gekennzeichnet,

daß Knoblauchextrakt eingesetzt ist, das aus kleingeschnittenem, getrocknetem, vorzugsweise gefriergetrocknetem, Knoblauch gewonnen ist.

6. Präparat nach Anspruch 1, dadurch gekennzeichnet,

daß Knoblauchextrakt eingesetzt ist, der durch Anwendung organischer Lösungsmittel aus frischem, kleingeschnittenen Knoblauch gewonnen ist.

7. Präparat nach Anspruch 1, dadurch gekennzeichnet,

daß Knoblauchextrakt eingesetzt ist, der durch Mazerisieren von frischem, kleingeschnittenen Knoblauch mit dem Fischöl gewonnen ist.

8. Präparat nach Anspruch 7, dadurch gekennzeichnet,

daß das Fischöl und der Knoblauchextrakt in dem Mengenverhältnis, in dem es nach der vorgenommenen Mazeration vorliegt, eingesetzt wird.

9. Präparat nach Anspruch 1,
gekennzeichnet durch die Form von Kapseln mit einem Fischölanteil von 500 bis 1000, vorzugsweise 750, mg (Milligramm) pro Kapsel.

**Claims**

1. Pharmaceutical preparation for the treatment of lipid metabolism disorders (hyperlipoproteinemia), which contains as the active agent garlic extract and highly unsaturated fatty acids of the omega-3 type, including eicosapentaenic acid, and oil as the carrier substance, characterized in that, based on the garlic extract obtained from 100 parts by weight of fresh garlic, it contains 50 to 200 and preferably 100 parts by weight of fish oil, obtained from the fish flesh of cold water fish, and highly unsaturated fatty acids of the omega-3 type and that the fish oil used contains 15 to 30% by weight of eicosapentaenic acid.

2. Preparation according to claim 1, characterized in that 0.5 to 2.0 and preferably 1.3 parts by weight of vitamin E are used on garlic extract from 100 parts by weight of fresh garlic.

3. Preparation according to claim 1, characterized in that the fish oil used contains 18 to 50 and preferably 22 to 31% by weight of highly unsaturated fatty acids of the omega-3 type.

4. Preparation according to claim 1, characterized in that the fish oil used contains 7 to 20 and preferably 10 to 13% by weight of docosahexaenic acid.

5. Preparation according to claim 1, characterized in that garlic extract is used, which is obtained from cut small, dried and preferably lyophilized garlic.

6. Preparation according to claim 1, characterized in that garlic extract is used, which is obtained from fresh, cut small garlic by using organic solvents.

7. Preparation according to claim 1, characterized in that garlic extract is used, which is obtained by the maceration of fresh, cut small garlic with fish oil.

8. Preparation according to claim 7, characterized in that the fish oil and garlic extract are used in the quantity ratio in which same are present following maceration.

9. Preparation according to claim 1, characterized in that each capsule contains 500 to 1000 and preferably 750 mg of fish oil.

**Revendications**

1. Préparation pharmaceutique pour le traitement de dérèglements du métabolisme des lipides ( hyperlipoprotéinémie) , qui contient comme substance active de l'extrait d'ail et des acides gras hautement insaturés de type oméga-3 ,parmi lesquels aussi l'acide eicosapentaénoique et de l'huile comme substance de support ou véhicule , préparation caractérisée en ce que , par rapport à l'extrait d'ail , qui a été obtenu à partir de 100 parties en poids d'ail frais ,la préparation contient 50 à 200 , avantageusement 100 ,parties en poids d'huile de poisson , qui a été obtenue à partir de chair de poissons d'eau froide et présente des acides gras hautement insaturés de type oméga-3 , et en ce que l'huile de poisson utilisée contient 15 à 30 % en poids d'acide eicosapentaénoïque .

2. Préparation selon la revendication 1 ,caractérisée en ce qu'on utilise 0,5 à 2,0 , avantageusement 1,3, parties en poids de vitamine E pour l'extrait d'ail provenant de 100 parties en poids d'ail frais .

3. Préparation selon la revendication 1 ,caractérisée en ce que l'huile de poisson utilisée contient 18 à 50 % en poids ,avantageusement 22 à 31 % en poids, d'acides gras hautement insaturés de type oméga-3 .

4. Préparation selon la revendication 1 , caractérisée en ce que l'huile de poisson utilisée contient 7 à 20 % en poids, avantageusement 10 à 13 % en poids d'acide docosahexaénoïque .

5. Préparation selon la revendication 1 , caractérisée en ce qu'on utilise de l'extrait d'ail qui a été obtenu à partir d'ail coupé en petits morceaux, séché, de préférence séché par congélation.

6. Préparation selon la revendication 1 , caractérisée en ce qu'on utilise de l'extrait d'ail qui a été obtenu à partir d'ail frais, coupé en petits morceaux et traité par appli cation d'un solvant organique.

7. Préparation selon la revendication 1 , caractérisée en ce qu'on utilise de l'extrait d'ail qui a été obtenu en faisant macérer de l'ail frais,coupé en petits morceaux ,avec de l'huile de poisson .

8. Préparation selon la revendication 7 , caractérisée en ce qu'on utilise l'huile de poisson et l'extrait d'ail en le rapport quantitatif existant après réalisation de la macération .

9. Préparation selon la revendication 1 , caractérisée en ce qu'elle est sous forme de capsules ou gélules contenant de 500 à 1 000 ,avantageusement 750 mg ( milligrammes) d'huile de poisson par capsule ou gélule .